# EUROPÄISCHE PATENTANMELDUNG

(11) **EP 2 327 390 A1**
(43) Veröffentlichungstag der Anmeldung: **01.06.2011**
(21) Anmeldenummer: 09014677.0
(22) Anmeldetag: 25.11.2009
(51) Int. Cl.: A61K 8/02, A61K 8/34, A61K 8/37, A61K 8/39, A61K 8/86, A61Q 19/10

(54) **Konzentrate zum Behandeln von Substraten**

(71) Anmelder: Cognis IP Management GmbH, 40589 Düsseldorf (DE)
(72) Erfinder: Schmitz, Jana, 40724 Hilden (DE); Schulte, Petra, 50733 Köln (DE); Kawa, Rolf, 40789 Monheim (DE); Stork, Anja, 50733 Köln (DE)

(57) **Zusammenfassung**

Die vorliegende Erfindung betrifft Zusammensetzungen, enthaltend
a. mindestens eine Verbindung ausgewählt aus der Gruppe bestehend aus
(a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
(a-2) Anlagerungsprodukten von 2 bis 50 Mol Ethylenoxid an Sorbitanmono- und/oder diester von gesättigten oder ungesättigten Fettsäuren mit 6 bis 22 C Atomen, und
(a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen.

b. mindestens einen ethoxylierten und/oder propoxylierten Glycerin Fettsäuremono-und/oder diester mit einem Ethoxylierungsgrad von kleiner 20, vorzugsweise 3 bis 19, insbesondere 3 bis 15,
c. mindestens ein Polyol ausgewählt aus der Gruppe bestehend aus Glycerin, Diglycerin, Triglycerin, Tetraglycerin, Alkylenglykolen und Polylalkylenglykolen,
d. mindestens ein Glycerin Fettsäuremonoester der allgemeinen Formel (I) 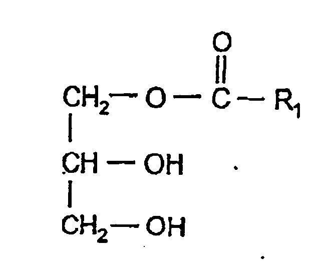 worin R₁ einen gesättigten oder ungesättigten, verzweigten oder lineare Rest mit 9 bis 19 C Atomen darstellt,
e. kleiner gleich 20 Gew.-% Wasser,

die Verwendung dieser Zusammensetzungen Substrate, die mit der Zusammensetzung behandelt sind.

## Beschreibung

Im Bereich der Pflege und Reinigung von Haut und Haar gewinnen feuchte und trockene Papiere oder Tücher immer größere Bedeutung. Der Begriff "Tücher" umfasst dabei die unterschiedlichsten Substrate, wie beispielsweise Vliese, Tissues und Papiere.

Unter dem Oberbegriff "Papier bzw. Tuch" werden ca. 3000 verschiedene Sorten und Artikel verstanden, die sich in ihren Anwendungsgebieten und ihrer Beschaffenheit zum Teil erheblich unterscheiden können. Zu ihrer Herstellung werden eine Reihe von Zusatzstoffen benötigt, von denen Füllstoffe (z.B. Kreide oder Kaolin) und Bindemittel (z.B. Stärke) zu den wichtigsten zählen. Für den Bereich der Tissue- und Hygienepapiere und -tücher, die in engeren Kontakt mit der menschlichen Haut gebracht werden, besteht ein besonderes Bedürfnis nach einem angenehmen Weichgriff, der dem Papier üblicherweise durch eine sorgfältige Auswahl der Faserstoffe und insbesondere einen hohen Anteil an frischem Holzschliff oder Cellulose verliehen wird.

Diese Papiere oder Tücher können mit den unterschiedlichsten Tränklösungen behandelt sein, die dem jeweiligen Papier oder Tuch seine pflegenden und/oder reinigenden Eigenschaften verleihen.

Eine Aufgabe der vorliegenden Erfindung bestand darin, eine Zusammensetzung bereit zu stellen, die bei Normaltemperatur fließfähig und somit leicht pumpbar ist. Gleichzeitig sollte diese Zusammensetzung stabil sein, d.h. es sollte keine Trennung der Bestandteile stattfinden. Weiterhin war erstrebenswert, dass diese Zusammensetzungen nach Verdünnen mit Wasser (sowie gegebenenfalls weiteren kosmetischen Inhaltsstoffen, insbesondere mit lipophilen Komponenten) keine Phasenseparation zeigen, von besonderem Interesse ist hier die Stabilität über einen längeren Zeitraum bei erhöhten Temperaturen. Weiterhin war von Interesse, dass sich diese Zusammensetzungen mit geringem Energieaufwand verdünnen lassen.

Weiterhin sollten die Zusammensetzungen gute sensorische Eigenschaften aufweisen bzw. dem Substrat, auf das sie aufgebracht werden, vorteilhafte sensorische Eigenschaften verleihen, hierbei war vor allen die Weichheit des mit der Zusammensetzung behandelten Substrats von Interesse. Es bestand weiterhin das Bedürfnis Zusammensetzungen bereit zu stellen, die es ermöglichen weitere Bestandteile, insbesondere öllösliche Bestandteile, auf das Substrat aufzubringen. Eine weitere Aufgabe bestand darin, Substrate zu Verfügung zu stellen, die eine hautschonende Reinigung der Haut ermöglichen und gleichzeitig eine hohe Hautverträglichkeit aufweisen. Hierbei waren vor allem die Rückfettung sowie die Feuchtigkeitsbindung der Haut von Interesse.

Die Aufgabe wird gelöst durch Zusammensetzungen enthaltend
a. mindestens eine Verbindung ausgewählt aus der Gruppe bestehend aus
   (a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
   (a-2) Anlagerungsprodukten von 2 bis 50 Mol Ethylenoxid an Sorbitanmono- und/oder diester von gesättigten oder ungesättigten Fettsäuren mit 6 bis 22 C Atomen, und
   (a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen,
b. mindestens einen ethoxylierten und/oder propoxylierten Glycerin Fettsäuremono-und/oder diester mit einem Ethoxylierungsgrad von kleiner 20, vorzugsweise 3 bis 19, insbesondere 3 bis 15,
c. mindestens ein von den Komponenten a, b und d verschiedenes Polyol,
d. mindestens ein Glycerin Fettsäuremonoester der allgemeinen Formel (l) 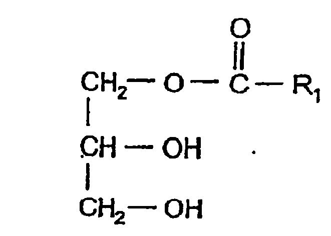 worin R₁ einen gesättigten oder ungesättigten, verzweigten oder lineare R mit 9 bis 19 C Atomen darstellt,
e. kleiner gleich 20 Gew.-% Wasser

Eine bevorzugte Ausführungsform der Erfindung betrifft Zusammensetzungen enthaltend
a. mindestens eine Verbindung ausgewählt aus der Gruppe bestehend aus
   (a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
   (a-2) Anlagerungsprodukten von 2 bis 50 Mol Ethylenoxid an Sorbitanmono- und/oder diester von gesättigten oder ungesättigten Fettsäuren mit 6 bis 22 C Atomen, und
   (a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen,
b. mindestens einen ethoxylierten und/oder propoxylierten Glycerin Fettsäuremono-und/oder diester mit einem Ethoxylierungsgrad von kleiner 20, vorzugsweise 3 bis 19, insbesondere 3 bis 15,
c. mindestens ein Polyol ausgewählt aus der Gruppe bestehend aus Glycerin, Diglycerin, Triglycerin, Tetraglycerin, Alkylenglykolen und Polylalkylenglykolen,
d. mindestens ein Glycerin Fettsäuremonoester der allgemeinen Formel (1) 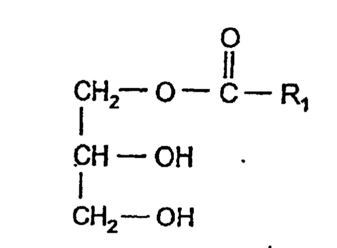 worin R₁ einen gesättigten oder ungesättigten, verzweigten oder lineare Rest mit 9 bis 19 C Atomen darstellt,
e. kleiner gleich 20 Gew.-% Wasser

Die erfindungsgemäßen Zusammensetzungen sind fließfähig. Als fließfähig werden solche Zusammensetzungen bezeichnet, deren Viskosität unterhalb 20 Pas (Brookfield Rotationsviskosimeter, RVF, Spindel TE, 4 UpM, 23 °C) liegt. In einer bevorzugten Ausführungsform der Erfindung sind die erfindungsgemäßen Zusammensetzungen dünnflüssig. Als dünnflüssig werden solche Zusammensetzungen bezeichnet, deren Viskosität kleiner gleich 800 mPas (Brookfield RVF 23°C, Spindel 5, 10UpM) beträgt.

Alle Gew.-% Angaben beziehen sich auf das Gesamtgewicht der Zusammensetzung

### Komponente (a)

Die Zusammensetzungen enthalten als Komponente (a) mindestens eine Verbindung ausgewählt aus der Gruppe bestehend aus
(a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
(a-2) Anlagerungsprodukten von 2 bis 50 Mol Ethylenoxid an Sorbitanmono- und/oder diester von gesättigten oder ungesättigten Fettsäuren mit 6 bis 22 C Atomen,
(a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen.

### Komponente (a-1)

Als Komponente (a) geeignet sind ethoxylierte und/oder propoxylierte Triglyceride der Ricinolsäure mit einem Ethoxylierungs- und/oder Propoxylierungsgrad von größer gleich 20, insbesondere von 20 bis 60. Diese Verbindungen können hydriert und/oder nicht hydriert sein.

Die ethoxylierten Triglyceride der Ricinolsäure folgen der allgemeinen Formel: 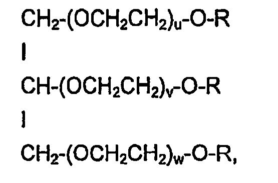 wobei
- u, v und w unabhängig voneinander eine Zahl von 0 bis 100 darstellt, und
- die Summe aus u, v und w größer gleich 20, vorzugsweise 20 bis 60 beträgt, und
- R den Acyl-Rest der Ricinolsäure [(*R*)-12-Hydroxy-(*Z*)-Octadec-9-ensäure] darstellt.

Die propoxylierten Triglyceride der Ricinolsäure folgen der folgenden Formel: 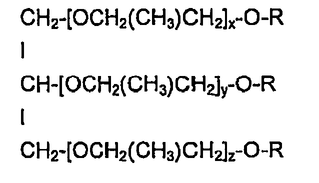 wobei
- x, y und z unabhängig voneinander eine Zahl von 0 bis 100 darstellt, und
- die Summe aus x, y und z größer gleich 20, vorzugsweise 20 bis 60, beträgt, und
- R den Acyl-Rest der Ricinolsäure [(*R*)-12-Hydroxy-(Z)-Octadec-9-ensäure] darstellt.

Erfindungsgemäß geeignet sind weiterhin Triglyceride der Ricinolsäure, die ethoxyliert und propoxyliert wurden: 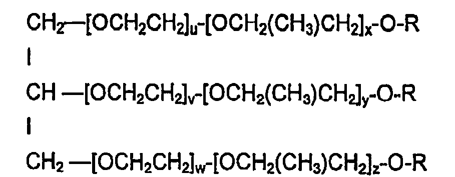 wobei
- u, v, w, x, y und z unabhängig voneinander eine Zahl von 0 bis 100 darstellt, und
- die Summe aus u, v, w, x, y und z größer gleich 20, vorzugsweise 20 bis 60, beträgt, und
- R den Acyl-Rest der Ricinolsäure [(*R*)-12-Hydroxy-(*Z*)-Octadec-9-ensäure] darstellt.

Die Reihenfolge der Ethylenoxid und Propylenoxid Einheiten in den ethoxylierten und propoxylierten Triglyceriden der Ricinolsäure hängt dabei von den bei der Herstellung gewählten Bedingungen ab.

In denen durch Hydrierung erhältlichen hydrierten oder teilhydrierten (ethoxylierten und/oder propoxylierten) Triglyceriden sind die Doppelbindungen in den Acyl-Resten der Ricinolsäure vollständig oder teilweise gesättigt.

Als Komponente (a) können sowohl ethoxylierte als auch propoxylierten bzw. die jeweils entsprechenden hydrierten Verbindungen einzeln oder in Gemischen untereinander eingesetzt werden.

Der Ethoxylierungs- bzw. Propoxylierungsgrad gibt die durchschnittlichen Mol Ethylenoxid bzw. Propylenoxid Einheiten an, und entspricht somit der Summe von u, v, w, x, y und z.

Die erfindungsgemäß als Komponenten (a-1) geeigneten Triglyceride der Ricinolsäure sind z.B. als Hauptbestandteil in kommerziell erhältlichen ethoxylierten Ricinusölen oder propoxylierten Ricinusölen enthalten. Diese sind kommerziell beispielsweise unter folgenden INCI Bezeichnungen erhältlich (in Klammern jeweils der Handelsname und Hersteller):
- INCI: PEG-20 Castor Oil (Nikkol CO-20TX, Nikko Chemical Co.)
- INCI: PEG-20 Hydrogenated Castor Oil (Nikkol HCO-20, Nikko Chemical Co.)
- INCI: PEG-40 Hydrogenated Castor Oil (Eumulgin^{®} HRE 40, Cognis GmbH; Cremophor RH 40, BASF Corp.; Tagat CH 40, Evonik Goldschmidt Corp.),
- IINCI: PEG-60 Hydrogenated Castor Oil (Eumulgin^{®} HRE 60, Cognis GmbH; Cremophor RH 60, BASF Corp.; Tagat CH 60, Evonik Goldschmidt Corp.; Croduret 60, Croda Europe)
- INCI: PEG-40 Castor Oil (Eumulgin^{®} RO 40, Cognis GmbH; Etocas 40, Croda Europe; Nikkol CO-40TX, Nikko Chemical Co.)
- INCI: PEG-60 Castor Oil (Handelsname Nikkol CO-60TX, Nikko Chemical Co.)
- INCI: PEG-80 Hydrogenated Castor Oil (Handelsname Nlkkol HCO-80, Nikko Chemical Co)
- INCI: PEG-100 Hydrogenated Castor Oil (Handelsname Nikkol HCO-100, Nikko Chemical Co)

### Komponente (a-2)

Als Komponente (a) weiterhin geeignet sind Anlagerungsprodukte von 2 bis 50 Mol Ethylenoxid an Sorbitanmono- und/oder diester von gesättigten oder ungesättigten Fettsäuren mit 6 bis 22 C Atomen.

In einer bevorzugten Ausführungsform werden Anlagerungsprodukte von 2 bis 50 Mol Ethylenoxid an Sorbitanmono- und/oder diester von gesättigten und linearen Fettsäuren mit 6 bis 22 C Atomen eingesetzt.

Sorbitan ist die Sammelbezeichnung für 4-wertige Alkohole, die durch Entzug von 1 Mol Wasser aus D-Glucitol (Sorbit) durch Erhitzen unter dem katalytischen Einfluss von Säuren entstehen. Durch Veresterung mit Fettsäuren enstehen die entsprechenden Sorbitanester (1,4 Sorbitanester sowie 1,5 Sorbitanester).

Erfindungsgemäß geeignete Sorbitanmonoester sind beispielsweise Verbindungen der folgenden Formel (ethoxylierte 1,4-Sorbitanmonoester): 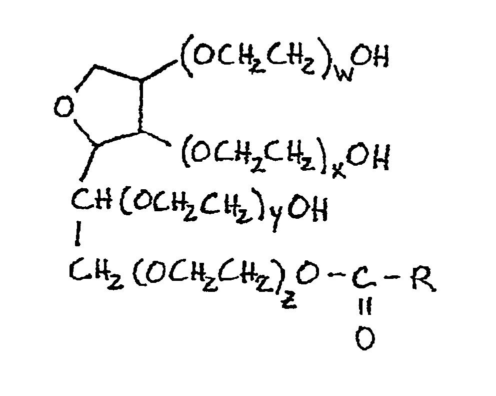
- in der R für einen gesättigten oder ungesättigten, linearen oder verzweigten Rest mit 5 bis 21 C Atomen steht
- in der w, x, y und z unabhängig voneinander für Zahlen von 0 bis 50 stehen,
- wobei die Summe aus w, x, y und z 2 bis 50 beträgt.

Besonders bevorzugt sind Verbindungen dieser Formel, in der R einen linearen und gesättigten Rest mit 11 bis 17 Kohlenstoffatomen darstellt.

Besonders bevorzugt sind Verbindungen dieser Formel, in der die Summe aus w, x, y und z 4 bis 40 beträgt.

Typische Beispiele für erfindungsgemäß geeignete ethoxylierte Sorbitanester sind die unter der INCI Bezeichnung Polysorbat 20 erhältlichen Produkte (Handelsname Eumulgin®SML 20, Cognis GmbH). Polysorbat 20 ist ein Gemisch von hauptsächlich Sorbitanmonoestem der folgenden Formel, in der die Summe aus w +x +y +z durchschnittlich 20 beträgt.

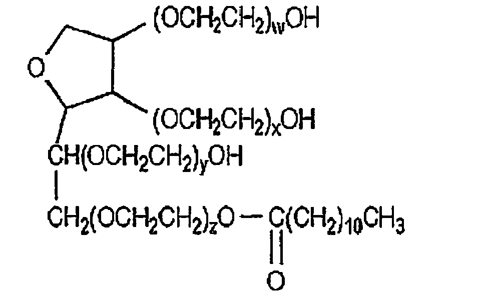

Weiterhin geeignete ethoxylierte Sorbitanester sind die unter der INCI Bezeichnung Polysorbat 21 erhältlichen Produkte (Handelsname Tween 21, Croda), bei denen die Summe aus w +x +y +z durchschnittlich 4 beträgt.

Weiterhin sind beispielsweise geeignet sind die unter der INCI Bezeichung Polysorbat 40 erhältlichen Produkte (Handelsname Tween 40, Croda). Polysorbat 40 ist ein Gemisch aus hauptsächlich Sorbitanmonoesters der folgenden Formel, in der die Summe aus w +x +y +z durchschnittlich 20 beträgt.

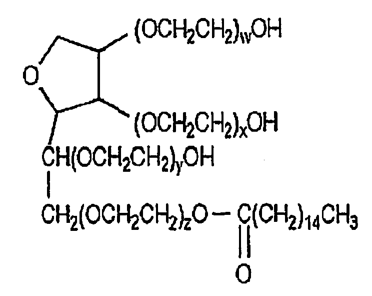

### Komponente (a-3)

Als Komponente (a) weiterhin geeignet sind Anlagerungsprodukte von 2 bis 50 Mol Ethylenoxid und 1 bis 20 Mol Propylenoxid an mit Polyolen ringgeöffneten alpha-Olefin-Epoxiden mit 8 bis 22 C Atomen.

Alpha-Olefin-Epoxide mit 8 bis 22 C Atomen sind Verbindungen, die endständig eine Epoxidgruppe tragen und der allgemeinen Formel folgen: 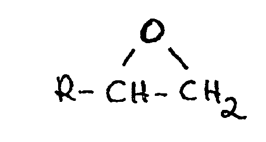 worin R einen Rest mit 6 bis 20 C Atomen darstellt.

Die Komponenten (a) werden erhalten durch Ringöffnung von alpha-Olefin Epoxiden mit 8 bis 22 C Atomen durch Polyole und anschließende Propoxylierung und Ethoxylierung (bzw. anschließende Ethoxlierung und Propoxylierung). Bei den Polyolen, die zur Ringöffnung verwendet werden handelt es sich vorzugsweise um Alkylenglykole oder Polyethylenglykole, insbesondere um Ethylenglykol.

In einer bevorzugten Ausführungsform der Erfindung wird als Komponente (a) eine Verbindung eingesetzt, die durch Ringöffnung von alpha-Olefin-Epoxiden mit 8 bis 22 C Atomen, vorzugsweise von alpha-Olefin-Epoxiden mit 8 bis 16, vorzugsweise mit 12 bis 16 C Atomen erhalten wird.

In einer bevorzugten Ausführungsform der Erfindung wird als Komponente (a) eine Verbindung eingesetzt, die durch Ringöffnung von alpha-Olefin-Epoxiden mit Ethylenglykol erhalten wird.

Diese Verbindungen folgen der allgemeinen Formel (I) und/oder (II):

(I) R-CH(OH)-CH₂-O-CH₂-CH₂-O-[CH(CH₃)CH₂-O]ₘ-[CH₂-CH₂-O]ₙ-H

worin
- m eine Zahl von 1 bis 20, vorzugsweise eine Zahl von 1 bis 5 darstellt
- n eine Zahl von 2 bis 50, vorzugsweise eine Zahl von 5 bis 30, insbesondere eine Zahl von 8 bis 25 darstellt
- R einen Rest mit 6 bis 20 C Atomen, vorzugsweise 10 bis 14 C Atomen darstellt

   (II) R-CH{-O[CH(-CH₃)CH₂-O]ₚ-[CH₂-CH₂-O]ₒ-H]}-CH₂-O-CH₂-CH₂-O-[CH(CH₃)CH₂-O]ₘ-[CH₂-CH₂-O]ₙ-H

   worin
- die Summe von m und p 1 bis 20, vorzugsweise 1 bis 5 beträgt
- die Summe von n und o 2 bis 50, vorzugsweise eine 5 bis 30, insbesondere eine Zahl von 8 bis 25 beträgt
- R einen Rest mit 6 bis 20 C Atomen, vorzugsweise 10 bis 14 C Atomen darstellt

Die Reihenfolge der Ethylenoxid- und Propylenoxideinheiten hängt dabei von den bei der Herstellung gewählten Reaktionsbedingungen ab. In einer bevorzugten Ausführungsform wird nach Ringöffnung des Epoxids zunächst eine Propoxylierung und dann eine Ethoxylierung durchgeführt. Diese Verbindungen entsprechen den Formeln (I) und/oder (II). Wird zunächst eine Ethoxylierung und daran anschließend eine Propoxylierung durchgeführt ist die Reihenfolge der Ethoxy- und Propoxyeinheiten in den Formeln (I) und (II) entsprechend vertauscht.

In einer bevorzugten Ausführungsform der Erfindung enthält die Zusammensetzung Verbindungen der allgemeinen Formel (I) und/oder (II), in der R einen Rest mit 10 C bis 14 C Atomen darstellt, m und p = 1, n und o = 9 betragen. Solche Verbindungen sind unter der INCI Bezeichnung PPG-1-PEG-9 Laurylglycolether (Eumulgin® L, Cognis GmbH) kommerziell erhältlich.

### Menge an (a)

In einer bevorzugten Ausführungsform der Erfindung enthalten die Zusammensetzungen 3 bis 50, insbesondere 5 bis 45, vorzugsweise 10 bis 40, insbesondere 20 bis 30 Gew.-% - bezogen auf das Gesamtgewicht der Zusammensetzung- der Komponente (a).

### Verhältnis von Komponente (b) zu (a)

In einer bevorzugten Ausführungsform der Erfindung ist das Gewichtsverhältnis von Komponente (b) zu (a) größer gleich 1, vorzugsweise größer gleich 1,5, insbesondere größer gleich 2. Vorzugsweise ist das Gewichtsverhältnis von (b) zu (a) größer gleich 5, insbesondere größer gleich 7, vorzugsweise größer gleich 10.

### Komponente (b)

Die Zusammensetzungen enthalten als Komponente (b) mindestens einen ethoxylierten und/oder propoxylierten Glycerin Fettsäuremono- und/oder -diester mit einem Ethoxylierungs- bzw. Propoxylierungsgrad von kleiner 20, vorzugsweise von 3 bis 19, insbesondere 3 bis 15.

Die Zusammensetzungen können als Komponente (b) sowohl einen ethoxylierten und/oder propoxylierten Glycerin Fettsäuremono- und/oder -diester mit einem Ethoxylierungs- bzw. Propoxylierungsgrad von kleiner 20 auch eine Mischung mehrerer ethoxylierter und/oder propoxylierter Fettsäuremono- und/oder -diester mit einem Ethoxylierungs- bzw. Propoxylierungsgrad von kleiner 20 enthalten.

Ethoxylierte und/oder propoxylierte Glycerin Fettsäuremono- und/oder -diester mit einem Ethoxylierungs- bzw. Propoxylierungsgrad von kleiner 20 folgend der allgemeinen Formel: 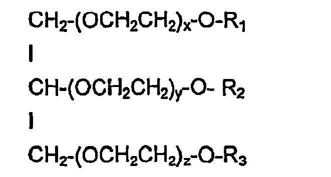 wobei
- x, y und z unabhängig voneinander eine Zahl von 0 bis 19 darstellt,
- die Summe von x, y und z 1 bis 19, vorzugsweise 3 bis 19, insbesondere 5 bis 15 beträgt,
- R₁, R₂ und R₃ unabhängig voneinander ausgewählt sind aus Wasserstoff, einem gesättigten oder ungesättigten, linearen oder verzweigten Acyl-Rest mit 8 bis 22 C Atomen, und
- mindestens ein Rest R₁, R₂ oder R₃ Wasserstoff darstellt.

Entsprechende folgen propoxylierte Glycerin-Fettsäuremono- und/oder diester mit einem Propoxylierungsgrad von kleiner 20 der folgenden allgemeinen Formel 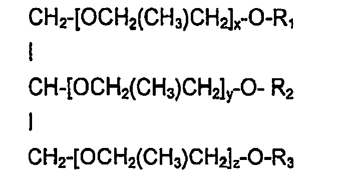 wobei
- x, y und z unabhängig voneinander eine Zahl von 0 bis 19 darstellt,
- die Summe von x, y und z 1 bis 19, vorzugsweise 3 bis 19, insbesondere 5 bis 15 beträgt,
- R₁, R₂ und R₃ unabhängig voneinander ausgewählt sind aus Wasserstoff, einem gesättigten oder ungesättigten, linearen oder verzweigten Acyl-Rest mit 8 bis 22 C Atomen, und
- mindestens ein Rest R₁, R₂ oder R₃ Wasserstoff darstellt.

Beispiele für geeignete Acyl-Reste R₁, R₂ oder R₃ sind
-C(=O)-rCH₂]₁₆ -CH₃ (= Octadecanoyl Rest, = Stearoyl Rest)
-C(=O)-[CH₂]₁₄ -CH₃ (= Hexandecanoyl Rest, = Palmitoyl Rest)
-C(=O)-[CH₂]₁₂ -CH₃ (= Tetradecanoyl Rest, = Myristoyl Rest)
-C(=O)-[CH₂]₁₀ -CH₃ (= Dodecanoyl Rest, = Lauroyl Rest)
-C(=O)-[CH₂]₈ -CH₃ (= Decanoyl Rest)
-C(=O)-[CH₂]₆ -CH₃ (= Octanoyl Rest)

Ebenso geeignet sind Glycerinfettsäuremonoester bzw. Glycerinfettsäurediester, die sowohl ethoxyliert als auch propoxyliert sind.

Als Komponente (b) können sowohl Glycerinfettsäuremonoester, Glycerinfettsäurediester als auch Mischungen aus Mono- und Diestern eingesetzt werden. Die erfindungsgemäß bevorzugt eingesetzten ethoxylierten/propoxylierten Glycerin-Fettsäureester sind in der Regel Gemische aus Glycerinfettsäuremonoestem und Glycerinfettsäurediestern.

Erfindungsgemäß bevorzugt kann als Komponenten (b) eine Verbindung eingesetzt werden, ausgewählt aus der Gruppe, die gebildet wird von
- Polyoxyethylen(10)olivenglyceride (INCI: PEG-10 Olive Glycerides),
- Polyoxyethylen(11)avocadoglyceride (INCI: PEG-11 Avocado Glycerides, CAS: 103819-44-9),
- Polyoxyethylen(11)kokusbuttergiyceride (INCI: PEG-11 Cocoa Butter Glycerides),
- Polyoxyethylen(9)kokusnussglyceride (INCI: PEG-9 Cocoglycerides, CAS: 67762-35-0),
- Mono-, Di- und Trihydrierte Palmkernglycerid PEG-6-Komplex (INCI: Hydrogenated Palm Kernel Glycerides PEG-6 Esters),
- Polyoxyethylen(7)glycerylmonococoat (CAS: 66105-29-1 und 68201-46-7) und
- Polyoxyethylen(6)caryl/capringlycerid (INCI: PEG-6 Caprylic/Capric Glycerides).

Dabei ist der Einsatz von Polyoxyethylen(7)glycerylmonococoat (CAS: 66105-29-1 und 68201-46-7), Polyoxyethylen(6)capryl/capringlycerid (INCI: PEG-6 Caprylic/Capric Glycerides) und Polyoxyethylen(9)kokusnussglyceride (INCI: PEG-9 Cocoglycerides, CAS: 67762-35-0) erfindungsgemäß besonders bevorzugt.

### Menge an (b)

In einer bevorzugten Ausführungsform der Erfindung enthalten die Zusammensetzungen 40 bis 90, insbesondere 50 bis 85, vorzugsweise 55 bis 80 Gew.-% - bezogen auf das Gesamtgewicht der Zusammensetzung- der Komponente (b).

### Komponente (c)

Die Zusammensetzungen enthalten als Komponente (c) mindestens ein Polyol. Bei der Komponente (c) handelt es sich dabei um eine von den Komponenten (a), (b) und (d) verschiedene Komponente.

Als Polyole im Sinne der Erfindung werden Substanzen verstanden, die mindestens zwei alkoholische Hydroxylgruppen im Molekül tragen. Vorzugsweise werden als Polyole Verbindungen eingesetzt, die 2 bis 15 Kohlenstoffatome und mindestens zwei Hydroxylgruppen tragen. Als Polyole können Verbindungen eingesetzt werden, die 3 bis 15 Kohlenstoffatome und mindestens drei Hydroxylgruppen tragen.

In einer bevorzugten Ausführungsform der Erfindung, enthalten die Polyole außer Sauerstoff, Wasserstoff und Kohlenstoff keine weiteren Elemente. Die Zusammensetzungen können als Komponente (c) sowohl ein Polyol als auch eine Mischung mehrerer Polyole enthalten.

In einer Ausführungsform der Erfindung sind die Polyole (c) ausgewählt aus der Gruppe bestehend aus Glycerin, Diglycerin, Triglycerin, Tetraglycerin, Alkylenglykolen und Polyalkylenglykolen.

Als **Alkylenglykole** geeignet sind Ethylenglycol (=1,2 Ethandiol), Diethylenglycol (=2,2'-Oxydiethanol; HO-(CH₂)₂-O-(CH₂)₂₋OH), Triethylenglycol (=2,2'-(Ethylendioxy)diethanol), 1,2-Propylenglycol, 1,3-Propylenglycol, Butylenglycole (=Butandiole) wie 1,2-Butylenglycol, 1,3 Butylenglycol, 1,4 Butyleneglycol, 2,3 Butyleneglycol; Pentan-1,5-diol; Pentan-1,2-diol; *meso*-Pentan-2,4-diol, (2R, 4R)-Pentan-2,4-diol; (2S, 4S)-Pentan-2,4-diol, Hexandiole, wie beispielsweise Hexylenglycol (= 2-Methylpentan-2,4-diol), Heptandiole, Octandiole und Decandiole.

Als Polyalkylenglycole werden überwiegend lineare, zum Teil aber auch verzweigte Polyether bezeichnet, die aus der Polykondensation von Glycolen entstehen. Die technisch wichtigen Vertreter dieser Polyether-Polyole sind die Polyethylenglycole, Polypropylenglycole, Polyethylene/Polypropyleneglycole sowie Polytetramethylenglycole bzw. deren analoge Verbindungen, die durch ringöffnende Polymerisation von Ethylenoxid, Propylenoxid bzw. Tetrahydrofuran hergestellt werden.

Besonders bevorzugt im Sinne der Erfindung sind als Polyalkylenglycole Polyethyleneglycole und/oder Polypropyleneglycole und/oder Polyethylen-Polypropyleneglycole mit einem durchschnittlichen Molekulargewicht von 100 bis 1000 Dalton.

Die bevorzugten Polyalkyleneglycole folgend dabei der allgemeinen Formel

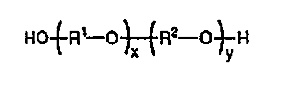

Sind R¹ und R² = (CH₂)₂ bezeichnet die Formel Polyethyleneglykole, sind R¹ und R² = CH₂-CH(CH₃) bezeichnet die Formel Polyproplyeneglykole. Ist R¹= (CH₂)₂ und R² = CH₂-CH(CH₃) bezeichnet die Formel Polyethylene/Polypropyleneglykole.

Dlese Verbindungen sind kommerziell beispielsweise unter der INCI Bezeichnung (in Klammer Handelsname und Hersteller) erhältlich:
INCI: PEG-4 (Polyglykol 200 USP, Clariant International; Pluracare E 200, BASF Corp.)
INCI: PEG-12 (Polyglykol 600, Clariant International; Pluracare E 600, BASF Corp.)
INCI: PPG-3 (Newpol PP-200, Sanyo Chemical Industries)

In einer weiteren Ausführungsform der Erfindung sind die Polyole (c) ausgewählt aus der Gruppe die gebildet wird von
- Glycerin, Diglycerin, Triglycerin, Tetraglycerin
- Alkylenglycolen ausgewählt aus der Gruppe bestehend aus Ethylenglycol (=1,2 Ethandiol), Diethylenglycol (=2,2'-Oxydiethanol; HO-(CH₂)₂-O-(CH₂)₂₋OH), Triethylenglycol (=2,2'-(Ethylendioxy)diethanol), 1,2-Propylenglycol, 1,3-Propylenglycol, Butylenglycole (=Butandiole) wie 1,2-Butylenglycol, 1,3 Butylenglycol, 1,4 Butyleneglycol, 2,3 Butyleneglycol; Pentan-1,5-diol; Pentan-1,2-diol; meso-Pentan-2,4-diol, (2R, 4R)-Pentan-2,4-diol; (2S, 4S)-Pentan-2,4-diol, Hexandiole, wie beispielsweise Hexylenglycol (= 2-Methylpentan-2,4-diol), Heptandiole, Octandiole und Decandiole.
- Polyalkyleneglycolen mit einem durchschnittlichen Molekulargewicht von 100 bis 1000 Dalton
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit
- kurzkettige Alkyglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### Menge an (c)

In einer bevorzugten Ausführungsform der Erfindung enthalten die Zusammensetzungen 1 bis 10, insbesondere 2 bis 8, bevorzugt 3 bis 6 Gew.-% - bezogen auf das Gesamtgewicht der Zusammensetzung- an Komponente (c).

### Komponente (d)

Die Zusammensetzungen enthalten als Komponente (d) mindestens ein Glycerin-Fettsäuremonoester der allgemeinen Formel 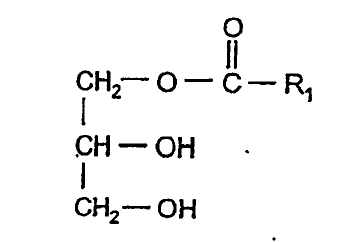 worin R₁ einen gesättigten oder ungesättigten, verzweigten oder linearen Rest mit 9 bis 19 C Atomen darstellt. Vorzugsweise stellt ist R ein linearer Rest, vorzugsweise ist R ein gesättigter Rest (Alkyl-Rest).

Beispiele für geeignete Glycerin-Fettsäuremonoester gemäß Formel (I) sind Glyceryl laurate [kommerziell erhältlich als Monomuls® 90-L 12, Cognis GmbH), Glyceryl myristate, Glyceryl stearate, Glyceryl oleate [kommerziell erhältlich als Monomuls® 90-O 18, Cognis GmbH), Glyceryl palmitate, Glyceryl palmitoleate, Glyceryl cocoate, Glyceryl linoloate.

In einer bevorzugten Ausführungsform der Erfindung wird als Glycerin-Fettsäuremonoester Glyceryl Oleat und/oder Glycerl Laurat (R₁ = ₙC₁₁H₂₃) eingesetzt.

Die erfindungsgemäßen Zusammensetzungen können als Komponente (d) sowohl ein Glycerin-Fettsäuremonoester als auch eine Mischung mehrerer Glycerin-Fettsäuremonoester enthalten.

### Menge an (d)

In einer bevorzugten Ausführungsform der Erfindung enthalten die Zusammensetzungen 0,5 bis 5, insbesondere 1 bis 3 Gew.-% - bezogen auf das Gesamtgewicht der Zusammensetzung an Komponente (d).

### Komponente (e)

Bei den erfindungsgemäßen Zusammensetzungen, wie beispielsweise die Zusammensetzungen gemäß Anspruch 1 und/oder 2, handelt es sich um "Konzentrate", d.h. die Zusammensetzungen enthalten kleiner gleich 20 kleiner gleich 20, insbesondere kleiner gleich 15, vorzugsweise kleiner gleich 10, insbesondere kleiner gleich 5 Gew.-% Wasser, bezogen auf das Gesamtgewicht der Zusammensetzung.

In einer besonders bevorzugten Ausführungsform der Erfindung enthält die Zusammensetzung kein zugesetztes Wasser und das in der Zusammensetzung enthaltene Wasser stammt ausschließlich aus dem Restwasser der Komponenten (a) bis (d). In diesem Fall liegt der Wassergehalt der Zusammensetzungen üblicherweise unter 3 Gew.-%, insbesondere unter 1 Gew.-%, bezogen auf das Gesamtgewicht der Zusammensetzung.

Eine bevorzugte Ausführungsform der Erfindung betrifft eine Zusammensetzung enthaltend
- 3 bis 50, insbesondere 5 bis 45, vorzugsweise 10 bis 40 Gew.-% mindestens einer Komponente (a)
- 40 bis 90, insbesondere 50 bis 85, vorzugsweise 55 bis 80 Gew.-% mindestens einer Komponente (b)
- 1 bis 10, insbesondere 2 bis 8, vorzugsweise 3 bis 6 Gew.-% mindestens einer Komponente (c)
- 0,5 bis 5, insbesondere 2 bis 8, vorzugsweise 3 bis 6 Gew.-% mindestens einer Komponente (d)
- kleiner gleich 20 Gew.-% Wasser

Alle Gewichtsangaben beziehen sich auf das Gesamtgewicht der Zusammensetzung.

Ein weiterer Gegenstand der Erfindung betrifft die Verwendung von Zusammensetzungen, die mindestens die folgenden Bestandteile enthält:
a. mindestens eine Verbindung ausgewählt aus der Gruppe bestehend aus
   (a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
   (a-2) Anlagerungsprodukten von 2 bis 50 Mol Ethylenoxid an Sorbitanmono- und/oder diester von gesättigten oder ungesättigten Fettsäuren mit 6 bis 22 C Atomen, und
   (a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen,
b. mindestens einen ethoxylierten und/oder propoxylierten Glycerin Fettsäuremono- und/oder diester mit einem Ethoxylierungsgrad von kleiner 20, vorzugsweise 3 bis 19, insbesondere 3 bis 15,
c. mindestens ein von den Bestandteilen a, b und d verschiedenes Polyol, vorzugsweise ein Polyol ausgewählt aus der Gruppe bestehend aus Glycerin, Diglycerin, Triglycerin, Tetraglycerin, Alkylenglykolen und Polylalkylenglykolen
d. mindestens ein Glycerin Fettsäuremonoester der allgemeinen Formel (I) 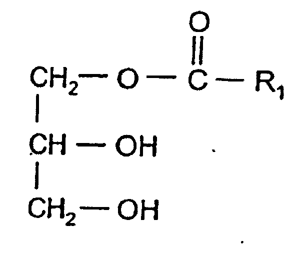
worin R₁ einen gesättigten oder ungesättigten, verzweigten oder lineare Rest mit 9 bis 19 C Atomen darstellt zum Behandeln von Substraten.

In einer Ausführungsform der Erfindung wird eine Zusammensetzung gemäß Anspruch 1 und/oder 2, gegebenenfalls nach Verdünnen mit Wasser und/oder weiteren Bestandteilen zum Behandeln von Substraten verwendet.

Der Begriff Behandeln umfasst dabei jede Art der Aufbringung einer Zusammensetzung auf mindestens eine Seite des Substrats. Hierzu eignen sich alle einschlägig bekannten Methoden, mit deren Hilfe man Flüssigkeiten auf mehr oder weniger feste Oberflächen auftragen kann. Beispielsweise seien genannt: Imprägnieren, Tränken, Beschichten, BeAnsprüchen, Tauchen, Ausrüsten, Abstreifen etc. Das Behandeln kann dabei bei Raumtemperatur oder unter Hitzeeinwirkung vorgenommen werden. Nach dem Auftragen der Zusammensetzungen kann sich ein kurzer Trockenschritt anschließen.

Die Zusammensetzungen und insbesondere die Konzentrate verleihen den mit Ihnen behandelten Substraten vorteilhafte pflegende bzw. sensorische Eigenschaften. Insbesondere verleihen die Zusammensetzungen und insbesondere die Konzentrate den mit Ihnen behandelten Substraten rückfettende und Feuchtigkeit spendende Eigenschaften. Mit den Zusammensetzungen und insbesondere mit den Konzentraten ist es weiterhin möglich, weitere öllösliche Substanzen auf die Substrate aufzubringen.

Ein weiterer Gegenstand der Erfindung betrifft ein **Verfahren zum Behandeln von Substraten,** bei dem man eine Zusammensetzung, die mindestens die folgende Bestandteile enthält:
a. mindestens eine Verbindung ausgewählt aus der Gruppe bestehend aus
   (a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
   (a-2) Anlagerungsprodukten von 2 bis 50 Mol Ethylenoxid an Sorbitanmono- und/oder diester von gesättigten oder ungesättigten Fettsäuren mit 6 bis 22 C Atomen, und
   (a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen,
b. mindestens einen ethoxylierten und/oder propoxylierten Glycerin Fettsäuremono-und/oder diester mit einem Ethoxylierungsgrad von kleiner 20, vorzugsweise 3 bis 19, insbesondere 3 bis 15,
c. mindestens ein von den Bestandteilen a, b und d verschiedenes Polyol, vorzugsweise ein Polyol ausgewählt aus der Gruppe bestehend aus Glycerin, Diglycerin, Triglycerin, Tetraglycerin, Alkylenglykolen und Polylalkylenglykolen
d. mindestens ein Glycerin Fettsäuremonoester der allgemeinen Formel (I) 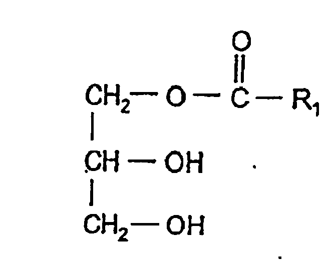
worin R₁ einen gesättigten oder ungesättigten, verzweigten oder lineare Rest mit 9 bis 19 C Atomen darstellt auf ein Substrat aufbringt und dieses gegebenenfalls trocknet.

Ein weiterer Gegenstand der Erfindung betrifft ein Verfahren zum Behandeln von Substraten, bei dem man eine Zusammensetzung nach Anspruch 1 und/oder 2, gegebenenfalls nach Verdünnen mit Wasser und weiteren Bestandteilen, auf ein Substrat aufträgt und dieses gegebenenfalls trocknet.

Die Zusammensetzungen nach Anspruch 1 und/oder 2 können unverdünnt auf das Substrat aufgebracht werden, üblicherweise werden sie jedoch verdünnt. Im einfachsten Fall erfolgt die Verdünnung mit Wasser, wobei die Zusammensetzung nach Anspruch 1 und/oder 2 üblicherweise in Mengen von 0,5 - 8,0 Gew.-%, vorzugsweise 0,5 - 5,0 Gew.-%, insbesondere 0,5 - 3,0 Gew.-% bezogen auf das Gesamtgewicht der Verdünnung eingesetzt wird.

Ein weiterer Gegenstand der Erfindung betrifft ein behandeltes Substrat, erhältlich, indem man auf ein Substrat mindestens die folgenden Bestandteile aufträgt:
a. mindestens eine Verbindung ausgewählt aus der Gruppe bestehend aus
   (a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
   (a-2) Anlagerungsprodukten von 2 bis 50 Mol Ethylenoxid an Sorbitanmono- und/oder diester von gesättigten oder ungesättigten Fettsäuren mit 6 bis 22 C Atomen, und
   (a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen,
b. mindestens einen ethoxylierten und/oder propoxylierten Glycerin Fettsäuremono-und/oder diester mit einem Ethoxylierungsgrad von kleiner 20, vorzugsweise 3 bis 19, insbesondere 3 bis 15,
c. mindestens ein von den Bestandteilen a, b und d verschiedenes Polyol, vorzugsweise ein Polyol ausgewählt aus der Gruppe bestehend aus Glycerin, Diglycerin, Triglycerin, Tetraglycerin, Alkylenglykolen und Polylalkylenglykolen
d. mindestens ein Glycerin Fettsäuremonoester der allgemeinen Formel (I) 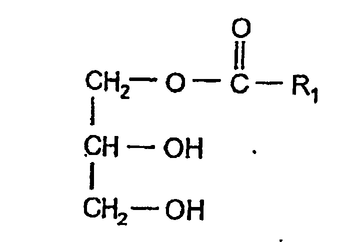
worin R₁ einen gesättigten oder ungesättigten, verzweigten oder lineare Rest mit 9 bis 19 C Atomen darstellt.

Die Bestandteile können dabei Einzeln oder in beliebigen Kombinationen auf das Substrat aufgebracht werden. In einer bevorzugten Ausführungsform der Erfindung werden die Bestandteile in Form einer Zusammensetzung bereitgestellt und das Substrat wird mit dieser Zusammensetzung behandelt. In einer bevorzugten Ausführungsform der Erfindung wird eine Zusammensetzung gemäß Anspruch 1 und/oder 2 bereitgestellt und direkt oder nach Verdünnen mit Wasser und gegebenenfalls weiteren Bestandteilen auf das Substrat aufgebracht. Daran kann sich ein Trocknungsschritt anschließen.

Ein weiterer Gegenstand der Erfindung betrifft ein behandeltes Substrat, erhältlich, indem man eine Zusammensetzung auf ein Substrat aufträgt, wobei die Zusammensetzung mindestens die folgenden Bestandteile enthält:
a. mindestens eine Verbindung ausgewählt aus der Gruppe bestehend aus
   (a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
   (a-2) Anlagerungsprodukten von 2 bis 50 Mol Ethylenoxid an Sorbitanmono- und/oder diester von gesättigten oder ungesättigten Fettsäuren mit 6 bis 22 C Atomen, und
   (a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen,
b. mindestens einen ethoxylierten und/oder propoxylierten Glycerin Fettsäuremono-und/oder diester mit einem Ethoxylierungsgrad von kleiner 20, vorzugsweise 3 bis 19, insbesondere 3 bis 15,
c. mindestens ein von den Bestandteilen a, b und d verschiedenes Polyol, vorzugsweise ein Polyol ausgewählt aus der Gruppe bestehend aus Glycerin, Diglycerin, Triglycerin, Tetraglycerin, Alkylenglykolen und Polylalkylenglykolen
d. mindestens ein Glycerin- Fettsäuremonoester der allgemeinen Formel (I) 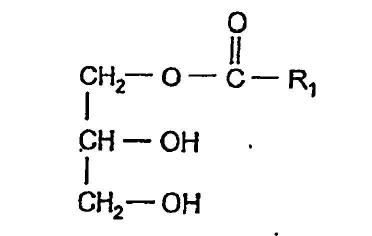
worin R₁ einen gesättigten oder ungesättigten, verzweigten oder lineare Rest mit 9 bis 19 C Atomen darstellt.

Ein weiterer Gegenstand der Erfindung betrifft ein behandeltes **Substrat,** erhältlich, indem man eine Zusammensetzung gemäß Anspruch 1 oder 2 auf ein Substrat aufträgt.

Die Bestandteile bzw. Zusammensetzungen verleihen den mit Ihnen behandelten Substraten vorteilhafte pflegende bzw. sensorische Eigenschaften. Insbesondere verleihen die sie den mit Ihnen behandelten Substraten rückfettende und Feuchtigkeit spendende Eigenschaften. Die so behandelten Substrate zeigen insbesondere bezüglich ihrer Weichheit gegenüber Substraten des Standes der Technik vorteilhaften Eigenschaften.

Die erfindungsgemäß behandelten Substrate können beispielsweise als Pflege - oder Reinigungstücher eingesetzt werden.

Dabei sind sowohl Anwendungen im Bereich der Hautpflege- oder Reinigung (insbesondere der Babypflege- oder Reinigung) möglich. Beispielsweise seien genannt Pflege- oder Reinigungstücher für die Gesichtshaut (sog. Facial tissues, Abschminktücher/Make-up Entfernung etc), Erfrischungstücher für die Haut, antibakterielle und/oder desodorierende Tücher, Produkte für die Intimpflege; wie beispielsweise Tampons, Damenbinden, Slipeinlagen, Intimpflegetücher), trockenes oder feuchtes Toilettenpapier, Inkontinenzprodukte, Selbstbräunungstücher oder sog. Insect Repellent Tücher. Mit den erfindungsgemäßen Zusammensetzungen ist es möglich die für die jeweilige Anwendung erforderlichen Bestandteile (z.B. desodorierende Wirkstoffe oder öllösliche Pflegekomponenten) auf das Substrat aufzubringen. Die so behandelten Substrate eignen sich weiterhin zur Desinfektion von Haut und Haar.

Dementsprechend kann man den Zusammensetzungen bzw. ihren Verdünnungen noch Parfüms, weitere Öle, Konservierungsmittel, UV-Lichtschutzfaktoren, biogene Wirkstoffe und Actives wie z.B. Dihydroxyaceton, Insect Repellents, antiinflammatorische Wirkstoffe (z.B. für Babytücher) oder Desinfektionsmittel zusetzen. Die Art und Mengen der jeweiligen Zusätze richten sich nach der beabsichtigten Verwendung.

Ein weiterer Gegenstand der Erfindung betrifft daher die Verwendung eines behandelten Substrats gemäß Ansprüchen 5 oder 6 zur Reinigung und/oder Pflege von Haut und Haar.

Ein weiterer Gegenstand der Erfindung betrifft daher die Verwendung eines behandelten Substrats gemäß Ansprüchen 5 oder 6 zur Desinfektion von Haut und Haar.

Als Substrat eignet sich prinzipiell jeglicher Träger, der es erlaubt die Zusammensetzung bzw. ihre Bestandteile aufzunehmen. Als Substrat geeignet sind beispielsweise Tissuepapiere und/oder Tissuegewebe und/oder Tissuetücher (im weiteren mit Tissuetücher bezeichnet). Diese können ein- oder mehrlagig aufgebaut sein. In der Regel weisen die Papiere ein Quadratmetergewicht von 10 bis 65, vorzugsweise 15 bis 30 g und eine Dichte von 0,6 g/cm und weniger auf. Beispiele für Tissuepapiere sind Toilettenpapiere, Papiertaschentücher, Gesichtsreinigungstücher, Abschminktücher, Erfrischungstücher, Haushaltstücher und dergleichen. Neben den papierbasierten Tissues kommen auch entsprechende Tissuegewebe in Frage, die aus Faser- oder Fleecestoff hergestellt werden.

Erfindungsgemäß als Substrat bevorzugt sind mehrlagige Tissuetücher. Insbesondere sind solche Tissuetücher bevorzugt, welche zwischen den einzelnen Lagen eine undurchlässige und/oder teildurchlässige Sperrschicht haben. Die teildurchlässige Sperrschicht kann beispielsweise als semipermeable Membran ausgebildet sein. Mit derartigen Tüchern können zwei oder mehrere Zusammensetzungen (gegebenenfalls nach vorheriger Verdünnung) auf ein Tuch aufgebracht werden. Dies kann ganz besonders bevorzugt sein, um mit der einen Seite der Tücher die Reinigung mittels der auf das Tuch aufgebrachten Zusammensetzung zu bewirken. Mit der anderen Seite kann dann beispielsweise zum Trocknen nach gerieben werden oder gegebenenfalls ein pflegender Wirkstoff auf die Haut aufgetragen werden.

Weiterhin kann es erfindungsgemäß ganz besonders bevorzugt sein, wenn die Tücher aus mindestens 3 Lagen mit Zusammensetzungen (gegebenenfalls nach vorheriger Verdünnung) behandelten Tissuetuches bestehen. Vorteilhaft ist dann zwischen mindestens 2 Lagen behandeltem Tuch jeweils 1 Lage Tuch als semipermeable Membran ausgebildet. Die semipermeable Membran ist dabei in Richtung der äußeren Tuchlagen durchlässig. Dadurch kann im Inneren beispielsweise eine Zusammensetzung (gegebenenfalls nach vorheriger Verdünnung) auf die innerste Schicht aufgebracht werden, welche entweder nicht mischbar und/oder nicht stabil mit der auf die äußere Seite aufgetragene Zusammensetzung ist. Hierdurch wird es möglich "two in one Tücher" zur Reinigung und Pflege anzubieten. Selbstverständlich umfasst die Erfindung die unterschiedliche farbliche Gestaltung der Tuchlagen. Weiterhin umfasst die erfindungsgemäße Lehre auch den Aufbau der Tücher aus mehreren Materialien, insbesondere in Bezug auf die Saugfähigkeit und Durchlässigkeit der unterschiedlichen Tuchlagen.

Als Substrat eignen sich weiterhin beispielsweise Textilfasern sowohl aus Naturfasern wie z.B. Cellulose, Seide, Wolle, Regeneratcellulose (Viskose, Rayon) Cellulosederivate als auch Textilfasern aus synthetischen Fasern wie z.B. Polyester-, Polypropylen-, Polyethylenterephtalat-, Polyamid-, Polyolefin-, Polyacrylnitril-Fasem oder Mischungen solcher Fasern. Dieser Fasern können gewebt oder ungewebt sein.

### Beispiele

Alle Gewichtsangaben in den Beispielen sind Gew.% Aktivsubstanz bezogen auf das Gesamtgewicht der Zubereitung

**Tabelle 1: Erfindungsgemäße Zusammensetzungen (Konzentrate)**

| | **Handelsname (INCI)** | **1** | **2** | **3** | **4** |
|---|---|---|---|---|---|
| (a) | Eumulgin^{®} HRE 40 (PEG-40 Hydrogenated Castor Oil)** | 30,0 | 28,5 | 28,5 | 5,0 |
| (b) | (PEG-7 Cocoglycerides) | 61,5 | 65,5 | 63,5 | 87,0 |
| (d) | Monomuts ^{®} 90-L 12 (Glyceryl Laurate) | | 3,0 | | |
| (d) | Monomuls^{®} 90-O 18 (Glyceryl Oleate) | 2,5 | | 3,0 | 3,0 |
| (c) | Propylene Glycol | 3,0 | | | |
| (c) | Glycerin | | 3,0 | 5,0 | 5,0 |
| (c) | Butylene Glycol | 3,0 | | | |
| | | | | | |
| | Aussehen des Konzentrates [RT] | klar | klar | klar | Klar |
| | Viskosität des Konzentrates [15°C] | dünn-flüssig* | dünn-flüssig* | dünn-flüssig* | dünn-flüssig* |
| | Viskosität des Konzentrates [RT] | dünn-flüssig* | dünn-flüssig* | dünn-flüssig* | dünn-flüssig* |
| | Stabilität des Konzentrates [15°C, RT & 40°C] nach 1 Woche | Stabil | Stabil | Stabil | Stabil |
| | Stabilität des Konzentrates [15°C, RT & 40°C] nach 8 Wochen | Stabil | Stabil | Stabil | Stabil |
| | Verdünnungen mit Wasser & Konservierungsmittel [RT & 40°C] | Stabil | Stabil | Stabil | Stabil |

| | | | | | |
|---|---|---|---|---|---|
| ** der Anteil an ethoxylierte Triglyceriden im Handelsprodukt Eumulgin® HRE 40 beträgt ca. 85 Gew.-%; RT = Raumtemperatur 23°C; *dünnflüssig: die Viskosität liegt unter 400 mPas (Brookfield RVT, 23°C, Spindel 5, 10UpM). | | | | | |

### Wässrige Verdünnungen der erfindungsgemäßen Zusammensetzungen

Aus den erfindungsgemäßen Zusammensetzungen 1 bis 3 (siehe Tabelle 1) wurden durch Zugabe von Wasser und gegebenenfalls weiteren Bestandteilen Verdünnungen hergestellt. Diese sind in den folgenden Tabellen aufgeführt:

| **Handelsname (INCI)** | **1** | **2** | **3** | **4** | **5** | **6** |
|---|---|---|---|---|---|---|
| Konzentrat 1, 2 oder 3 gemäß Tab. 1 | 1,0 | 2,0 | 4,0 | 1,5 | 3,0 | 1,5 |
| Uniphen^{®} P 23 | | | | 0,2 | | 0,2 |
| Nipaguard^{®} BPX | | 0,5 | | | | |
| Sodium Benzoate | 0,5 | | | 0,5 | | 0,5 |
| Euxyl PE^{®} 9010 | | | 1,0 | | | |
| Nipaguard® PO 5 | | | | | 1,0 | |
| Helianthus Annuus Seed Oil | 0,1 | | | | | |
| Cetiol® 868 (Ethylhexyl Stearate) | | | | 0,1 | | |
| Cetiol® Sensoft (Propylheptyl Caprylate) | | | | | | 0,1 |
| Cetiol® SN (Cetearyl lsononanoate) | | | 0,1 | | | |
| Propylene Glycol | | | | 1,0 | | 1,0 |
| Eumulgin® HRE 40 (PEG-40 Hydrogenated Castor Oil | | | | | | 0,8 |
| Parfüm | 0,1 | | | 0,1 | | 0,1 |
| Wasser | | | | | | |
| pH-Wert (ggf. mit pH-Adjuster einstellen) | 4,5-5,5 | 6,0-6,5 | 6,0- 6,5 | 4,5-5,5 | 5,0-6,5 | 4,5-5,5 |
| Stabiliät der Verdünnung [RT] | stabil | stabil | stabil | stabil | stabil | Stabil |

| **Handelsname (INCI)** | **7** | **8** | **9** | **10** | **11** |
|---|---|---|---|---|---|
| Konzentrat 1, 2 oder 3 gemäß Tab. 1 | 3,5 | 2,0 | 4,5 | 0,5 | 2,0 |
| Uniphen^{®} P 23 | | | 1,0 | | |
| Nipaguard^{®} BPX | | | | | 0,5 |
| Sodium Benzoate | | 0,5 | | 0,5 | |
| Sensiva SC 10 | 0,8 | | | | |
| Eumulgin^{®} HRE 40 (PEG-40 Hydrogenated Castor Oil) | | | 1,0 | | |
| Cosmedia^{®} SP (Sodium Polyacrylate) | | | | | 0,1 |
| Copherol^{®} 1250 C (Tocopheryl Acetate) | 0,1 | | | | 0,1 |
| Dihydroxyacetone (DHA) | | 2,0 | | | |
| Insect Repellent 3535^{®} (Ethyl Butylacetylaminopropionate) | | | 10,0 | | |
| Herbalis^{®} G Ginkgo | | | | 0,2 | |
| Perfume | | | | 0,1 | |
| Wasser | | | | | |
| pH-Wert (ggf. mit pH- Adjuster einstellen) | 6,0-6,5 | 3,5-4,0 | 6,0-6,5 | 4,5- 5,5 | 6,0- 6,5 |
| Stabiliät der Verdünnung [RT] | stabil | stabil | stabil | stabil | Stabil |

Alle Verdünnungen (jeweils mit Konzentrat 1, 2 oder 3) waren bei Raumtemperatur stabil. Die Konzentrate 1 bis 3 bzw. die daraus gemäß Tabelle 2 hergestellten Verdünnungen können auf die Substrate aufgetragen werden.

Folgende Tab. zeigt weitere Verdünnungen der erfindungsgemäßen Zusammensetzungen

| **Handelsname (INCI)** | **12** | **13** | **14** | **15** | **16** | **17** |
|---|---|---|---|---|---|---|
| Konzentrat 3 gemäß Tab. 1 | 1,0 | 1,5 | 1,0 | 1,0 | 1,0 | 1,0 |
| Cetiol®868 (Ethylhexylstearate) | 0,1 | 0,1 | - | - | - | - |
| Eutanol®G (Octyldodecanol) | - | - | - | - | - | 0,1 |
| Dehyton® DC (Disodium Cocoamphodiacetate) | - | - | 0,5 | - | - | - |
| HYDAGEN® C.A.T. (Triethyl Citrate) | - | - | 0,5 | - | - | - |
| ELESTAB® HP 100 (Hexamidine diisethionate) | - | - | - | - | - | 0,1 |
| Panthenol | 0,5 | - | - | - | - | - |
| Hydagen®B (Bisabolol) | - | 0,5 | - | - | - | - |
| Aloe Extract (Aloe Barbadensis) | - | - | - | - | 0,1 | - |
| Konservierungsmittel | qs | qs | qs | - | qs | - |
| Parfum | - | qs | qs | - | qs | qs |
| Isopropanol | - | - | - | 5,0 | - | - |
| Cetylpyridonium Chloride | - | - | - | 0,1 | - | 0,1 |
| KOH (1 %) | - | - | - | qs | - | qs |
| Wasser | 98,4 | 97,9 | 97,2 | 98,7 | 98,8 | 98,7 |
| Viscosity [mPas] (Brookfield RVF spindle 4, 10 rpm, 23°C) | <100 | <100 | <100 | <100 | <100 | <100 |
| pH-Wert (ggf. mit pH- Adjuster einstellen) | <5.5 | <5.5 | <5.5 | <5.5 | <5.5 | <5.5 |
| Stabiliät der Verdünnung [RT] | stabil | stabil | stabil | stabil | stabil | Stabil |

Die Verdünnungen 12, 13 und 14 eignen sich insbesondere zum Behandeln von Substraten, die dann zur Reinigung der Haut eingesetzt werden. Die Verdünnungen 15 und 17 eignen sich insbesondere zum Behandeln von Substraten, die dann zur Desinfektion der Haut eingesetzt werden. Die Verdünnung 16 eignet sich insbesondere zum Behandeln von Substraten, die dann als Intimpflegeprodukte eingesetzt werden.

Appendix: Eingesetzte Konservierungsstoffe und Booster: Handelsname und INCI Bezeichnung

**Uniphen^{®} P 23 INCI:** Phenoxyethanol, Methyparaben, Ethylparaben, Propylparaben, Butylparaben, Isobutylparaben; **Nipaguard^{®} BPX INCI:** Phenoxyethanol, Methylparaben, Propylparaben, 2-Bromo-2-Nitropropane-1,3-Diol; **Euxyl PE^{®} 9010** INCI: Phenoxyethanol, Ethylhexylglycerin; **Nipaguard® PO 5** INCI: Phenoxyethanol, Piroctone Olamine; **Sensiva SC 10** INCI: Caprylyl Glycol, Ethylhexylglycerin).

### Beispiel: Substrat behandelt mit erfindungsgemäßer Zusammensetzung.

Ein dreilagiger Vliesstoff der Fa. Pelytex besteht außen aus 22 grams per square metre Polypropylen und innen aus Vskose/Polypropylen (75/25; 80 grams per square metre). Ein Ausschnitt von 18 x 14 cm wird mit 1,5 g des Konzentrates gemäß Beispiel 1 (Tabelle 1) einseitig besprüht.

## Patentansprüche

1. Zusammensetzung enthaltend
a. mindestens eine Verbindung ausgewählt aus der Gruppe bestehend aus
(a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
(a-2) Anlagerungsprodukten von 2 bis 50 Mol Ethylenoxid an Sorbitanmono- und/oder diester von gesättigten oder ungesättigten Fettsäuren mit 6 bis 22 C Atomen, und
(a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen.
b. mindestens einen ethoxylierten und/oder propoxylierten Glycerin Fettsäuremono-und/oder diester mit einem Ethoxylierungsgrad von kleiner 20, vorzugsweise 3 bis 19, insbesondere 3 bis 15,
c. mindestens ein Polyol ausgewählt aus der Gruppe bestehend aus Glycerin, Diglycerin, Triglycerin, Tetraglycerin, Alkylenglykolen und Polylalkylenglykolen,
d. mindestens ein Glycerin Fettsäuremonoester der allgemeinen Formel (I) 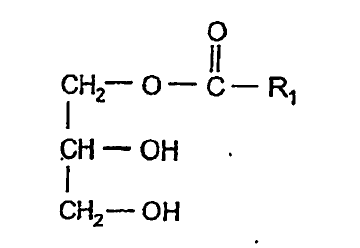 worin R₁ einen gesättigten oder ungesättigten, verzweigten oder lineare Rest mit 9 bis 19 C Atomen darstellt,
e. kleiner gleich 20 Gew.-% Wasser

2. Zusammensetzung nach Anspruch 1 enthaltend
- 3 bis 50, insbesondere 5 bis 45, vorzugsweise 10 bis 40 Gew.-% mindestens einer Komponente (a)
- 40 bis 90, insbesondere 50 bis 85, vorzugsweise 55 bis 80 Gew.-% mindestens einer Komponente (b)
- 1 bis 10, insbesondere 2 bis 8, vorzugsweise 3 bis 6 Gew.-% mindestens einer Komponente (c)
- 0,5 bis 5, insbesondere 2 bis 8, vorzugsweise 3 bis 6 Gew.-% mindestens einer Komponente (d)
- kleiner gleich 20 Gew.-% Wasser

3. Verwendung einer Zusammensetzung, die mindestens die folgende Bestandteile enthält:
a. mindestens eine Verbindung ausgewählt aus der Gruppe bestehend aus
(a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
(a-2) Anlagerungsprodukten von 2 bis 50 Mol Ethylenoxid an Sorbitanmono- und/oder diester von gesättigten oder ungesättigten Fettsäuren mit 6 bis 22 C Atomen, und
(a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen.
b. mindestens einen ethoxylierten und/oder propoxylierten Glycerin Fettsäuremono-und/oder diester mit einem Ethoxylierungsgrad von kleiner 20, vorzugsweise 3 bis 19, insbesondere 3 bis 15,
c. mindestens ein Polyol ausgewählt aus der Gruppe bestehend aus Glycerin, Diglycerin, Triglycerin, Tetraglycerin, Alkylenglykolen und Polylalkylenglykoien
d. mindestens ein Glycerin Fettsäuremonoester der allgemeinen Formel (I) 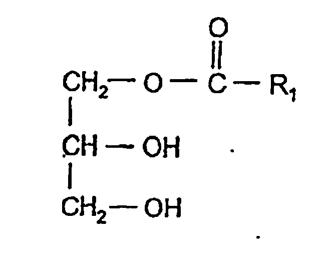 worin R₁ einen gesättigten oder ungesättigten, verzweigten oder lineare Rest mit 9 bis 19 C Atomen darstellt zum Behandeln von Substraten.

4. **Verfahren zum Behandeln von Substraten,** bei dem man eine Zusammensetzung, die mindestens die folgende Bestandteile enthält:
a. mindestens eine Verbindung ausgewählt aus der Gruppe bestehend aus
(a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
(a-2) Anlagerungsprodukten von 2 bis 50 Mol Ethylenoxid an Sorbitanmono- und/oder diester von gesättigten oder ungesättigten Fettsäuren mit 6 bis 22 C Atomen, und
(a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen.
b. mindestens einen ethoxylierten und/oder propoxylierten Glycerin Fettsäuremono-und/oder diester mit einem Ethoxylierungsgrad von kleiner 20, vorzugsweise 3 bis 19, insbesondere 3 bis 15,
c. mindestens ein Polyol ausgewählt aus der Gruppe bestehend aus Glycerin, Diglycerin, Triglycerin, Tetraglycerin, Alkylenglykolen und Polylalkylenglykolen
d. mindestens ein Glycerin Fettsäuremonoester der allgemeinen Formel (I) 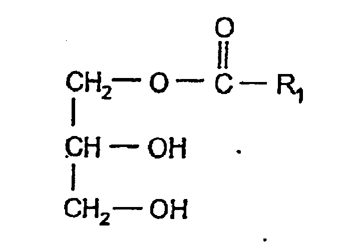 worin R₁ einen gesättigten oder ungesättigten, verzweigten oder lineare Rest mit 9 bis 19 C Atomen darstellt auf ein Substrat aufbringt und dieses gegebenenfalls trocknet.

5. **Behandeltes Substrat,** erhältlich, indem man auf ein Substrat mindestens die folgende Bestandteile aufträgt:
a. mindestens eine Verbindung ausgewählt aus der Gruppe bestehend aus
(a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
(a-2) Anlagerungsprodukten von 2 bis 50 Mol Ethylenoxid an Sorbitanmono- und/oder diester von gesättigten oder ungesättigten Fettsäuren mit 6 bis 22 C Atomen, und
(a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen
b. mindestens einen ethoxylierten und/oder propoxylierten Glycerin Fettsäuremono-und/oder diester mit einem Ethoxylierungsgrad von kleiner 20, vorzugsweise 3 bis 19, insbesondere 3 bis 15,
c. mindestens ein Polyol ausgewählt aus der Gruppe bestehend aus Glycerin, Diglycerin, Triglycerin, Tetraglycerin, Alkylenglykolen und Polylalkylenglykolen
d. mindestens ein Glycerin Fettsäuremonoester der allgemeinen Formel (I) 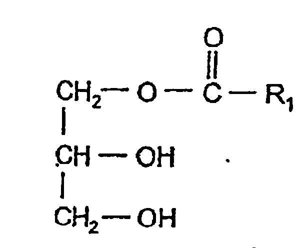 worin R₁ einen gesättigten oder ungesättigten, verzweigten oder lineare Rest mit 9 bis 19 C Atomen darstellt.

6. Behandeltes Substrat nach Anspruch 5, erhältlich indem man auf eine Substrat eine Zusammensetzung aufbringt, wobei die Zusammensetzung mindestens die folgenden Bestandteile enthält
a. mindestens eine Verbindung ausgewählt aus der Gruppe bestehend aus
(a-1) hydrierten und/oder nicht hydrierten, ethoxylierten und/oder propoxylierten Triglyceriden der Ricinolsäure mit einem Ethoxylierungs- und/oder Propoxylierungsgrad von größer gleich 20, insbesondere von 20 bis 60,
(a-2) Anlagerungsprodukten von 2 bis 50 Mol Ethylenoxid an Sorbitanmono- und/oder diester von gesättigten oder ungesättigten Fettsäuren mit 6 bis 22 C Atomen, und
(a-3) Anlagerungsprodukten von 2 bis 50 Mol Ethylenoxid und 1 bis 20 Mol Propylenoxid an mit Polyolen ringgeöffneten alpha-Olefin-Epoxiden mit 8 bis 22 C Atomen.
b. mindestens einen ethoxylierten und/oder propoxylierten Glycerin Fettsäuremono-und/oder diester mit einem Ethoxylierungsgrad von kleiner 20, vorzugsweise 3 bis 19, insbesondere 3 bis 15,
c. mindestens ein Polyol ausgewählt aus der Gruppe bestehend aus Glycerin, Diglycerin, Triglycerin, Tetraglycerin, Alkylenglykolen und Polyialkylenglykolen
d. mindestens ein Glycerin Fettsäuremonoester der allgemeinen Formel (I) 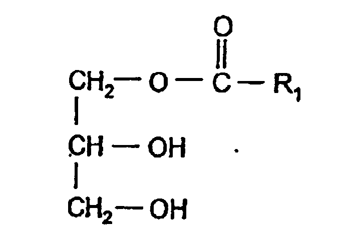 worin R₁ einen gesättigten oder ungesättigten, verzweigten oder lineare Rest mit 9 bis 19 C Atomen darstellt.

7. Verwendung eines Substrates gemäß Anspruch 5 oder 6 zur Reinigung und/oder Pflege von Haut und Haar.
